# EUROPEAN PATENT SPECIFICATION

(11) **EP 2 299 877 B1**
(45) Date of publication and mention of the grant of the patent: **02.01.2013**
(21) Application number: 09758799.2
(22) Date of filing: 02.02.2009
(51) Int. Cl.: A47G 9/02, A61F 7/00, A61F 7/03

(54) **WARMING BLANKETS, COVERS, AND APPARATUS, AND METHODS OF FABRICATING AND USING THE SAME**
WÄRMDECKEN, -ABDECKUNGEN UND -VORRICHTUNG UND VERFAHREN ZU DEREN HERSTELLUNG UND VERWENDUNG
COUVERTURES CHAUFFANTES, COUVERTURES, ET APPAREIL, ET PROCÉDÉS DE FABRICATION ET D'UTILISATION DE CELLES-CI

(30) Priority: 03.06.2008 US 58283 P; 13.06.2008 US 61404 P
(43) Date of publication of application: 30.03.2011
(73) Proprietor: Apricity Llc, Alpine, UT 84004 (US)
(72) Inventor: VERGONA, Joseph, Blase, Suwanee GA 30024 (US); KYKER, Mark, Uel, Carmel IN 46032 (US)
(74) Representative: Phillips & Leigh
(86) International application number: PCT/US2009/032868
(87) International publication number: WO 2009/148636

(56) References cited:
- WO-A-03/000564
- GB-A- 2 401 055
- US-A- 4 969 459
- US-A1- 2006 057 917
- US-A1- 2006 135 016
- US-A1- 2007 284 356

## Description

This application claims priority to both U.S. Serial No. 61/058,283, entitled "METHOD AND APPARATUS FOR PATIENT WARMING", filed June 3, 2008; and to U.S. Serial No. 61/061,404, entitled "WARMING BLANKETS AND METHODS OF FABRICATING THE SAME", filed June 13, 2008.

### FIELD OF THE INVENTION

Embodiments of the invention relate generally to warming blankets and, more specifically, to warming blankets, covers, and apparatus and methods of fabricating and using the same.

### BACKGROUND OF THE INVENTION

Conventional blankets are utilized in association with a wide variety of different applications and activities, for instance, recreational uses, outdoor uses, medical uses, etc. Such blankets are typically utilized in order to provide heat and/or protection to a user or group of users. In some instances, one or more electrical heating elements can be incorporated into such blankets in order to provide additional heat or comfort. These electrical heating elements are typically powered by an electrical power source. The portability of such blankets is often limited when the electrical heating elements are activated. In some instances, certain conventional blankets did not provide an adequate level of comfort in certain situations due to bulk or lack of performance as some users, such as medical patients, complained of such deficiencies. Additionally, safety considerations can arise when using such blankets due to environmental conditions including the presence of moisture or due to electromagnetic fields generated by such blankets. Therefore, it is often not feasible to utilize these conventional blankets in certain environments or in certain applications.

In some circumstances, certain persons have a need to maintain or otherwise regulate their body warmth. Humans have a very tight control of thermoregulation with the ability to maintain within a thermoregulatory threshold of approximately 0.2 °C to maintain a targeted thermal value. This tight threshold may be reduced in the elderly or the sick. The primary autonomic defenses of thermal challenge are sweating and thermoregulatory vasoconstriction that occurs primarily in the distal extremities, such as fingers and toes. This shunting of blood allows the core or central temperature to maintain a temperature of approximately 3°C to approximately 4°C warmer than the periphery (arms and legs). Shivering is the last autonomic defense that generates approximately 2 times to approximately 3 times the normal metabolic thermal production.

On induction of general anesthesia or major conduction anesthesia the normal thermal preventive measures are inhibited. The thermoregulation interthreshold (typically about 0.2 °C) range is widened by approximately 20 times so as to prevent the maintenance of normothermia. Obviously all behavioral modifications are prevented with anesthesia and the resultant loss of tonic vasoconstriction in the fingers and toes causes the redistribution of heat from the core to the periphery at a loss of approximately 0.6 °C per hour for about the first two hours. Approximately 90 percent of all heat is lost via the skin surface due to the vasodilation with only a small amount of loss via radiant and convective means. This loss will continue after the first two hours but at a reduced rate, primarily from continued heat loss pathways that exceed heat production as the metabolic rate is decreased under anesthesia. Eventually, reestablishment of thermoregulation will occur but only after significant hypothermia with the below mentioned comorbidities.

One conventional method to warm a hypothermic (below about 36°C) patient intraoperatively is with a forced air warmer. Use of blankets, convective covers, heated water mattresses, and fluid warming generally do not result in significant warming of an anesthetized patient.

Maintenance of normothermia is of clinical importance as well as patient satisfaction and may have an effect on payment for services for both the practitioner and the facility. Conventional active warming devices can include a forced air warmer that consists of a stand alone machine that has an electrical cord, corrugated tubing that affixes to the plastic disposable sheet that is placed over the patient in the operating room. The controls can be placed on a separate device that generates the warm flow or air that blows through the sheet. Most hospitals and surgery centers utilize this only in the operating room as it can require multiple machines to provide flow in each patient care space. The device itself can be somewhat cumbersome, and has cords that are intrusive especially in the preoperative and post operative site. At some or all settings the forced air warmer produces significant noise within the environment.

Patients undergoing anesthesia, general, neuraxial and even sedation will experience varying degrees of hypothermia (becoming cold). This effect is most pronounced during about the first two hours of anesthesia. Typically this loss of core temperature occurs at about 0.6°C per hour for about the first two hours. This resulting unintentional anesthetic induced mild hypothermia has many well documented ill effects, some of which include an increase in surgical wound infections, coagulopathy, increased cardiac morbidity, prolonged emergence and recovery translating into longer hospital stays and increased costs.

In fact, maintenance of normothermia is one of the proposed performance measures put forth by the American Society of Anesthesiologists. This is especially important since the AMA signed the Joint House-Senate Working Agreement that promised to develop about 140 performance measures covering about 34 clinical areas and, by 2007, that physicians would voluntarily report three to five measures each. Each of these measures has significant bearing on the practice of anesthesia as governmental pressures mount to pursue value-based purchasing (e.g., Deficit Reduction Act, Tax Relief Act) and potential financial incentives (Pay for Performance).

Mild hypothermia is often defined as about 34 to about 36 °C as measured at a core site (nasopharyx, esophageal, pulmonary artery, tympanic membrane thermocouple) and often will begin early in the preoperative period. Patients presenting for procedures are often anxious and then asked to disrobe, placed in a cool room wearing only a gown. Although many patients often complain of thermal discomfort during this preoperative time, there is little done other than providing passive warming with blankets. There have been studies that show significant improvement in the maintenance of normothermia by providing active warming in the preoperative period.

There are at least four types of heat loss pathways described as they relate to the perioperative period. These are evaporative, convective, radiant and conductive. All of these pathways work against normothermia in the perioperating arena. To combat these types of losses, humans have behavioral and autonomic responses to prevent hypothermia. When the patient enters the cold environment of the operating room he or she is unable to perform the normal behavioral response to thermal discomfort which consists of adding clothes or turning up the temperature in the room. On induction of anesthesia the autonomic function is inhibited.

Not to be forgotten is the thermal discomfort that many patients complain of during the perioperative process. As described above, this process often begins on admission for the procedure and can continue well into the recovery period. Although the warm blankets placed on the patient have an initial satisfaction, studies have shown the effect is brief. In summary, maintenance of perioperative normothermia has significant clinical importance as well as patient satisfaction issues, and may have an effect on payment for services for both the practitioner and the facility.

Because of the requirement for multiple machines, most facilities do not utilize the previously described forced air active warming device preoperatively and post operatively as a routine device. A forced air device is rarely found in the office based setting where about 25% of all elective procedures are now performed.

Currently the only active heating devices used in the perioperative arena are conventional forced air warmers.

Accordingly, there exists a need for warming blankets, covers, and apparatus, and methods of fabricating and using the same.
US 2007/284356 A1 discloses a warming blanket according to the preambles of claims 1 and 8.

### SUMMARY OF THE INVENTION

The present invention is as set out in the appended claims.

Embodiments of the invention can address some or all of the above needs. Certain embodiments of the invention can provide warming blankets, covers, and apparatus, and methods of fabricating and using the same.

In one embodiment, a warming blanket is provided. The warming blanket includes a body; and one or more pockets removably connected to the body, wherein each of the one or more pockets is adapted to hold a respective removable warming element.

In one aspect of the embodiment, each of the respective warming elements comprises carbon, charcoal, vermiculite, or an air-activated chemical composition.

In one aspect of the embodiment, the body has one of a substantially rectangular, a substantially square, a substantially elliptical, a substantially circular shape, or substantially conforms to the shape of a human body.

In one aspect of the embodiment, the body comprises one or more cut-out portions associated with one or more respective appendages of the human body.

In one aspect of the embodiment, the one or more pockets are connected to the blanket in accordance with a predetermined configuration.

In one aspect of the embodiment, a warming area provided by the one or more removable warming elements is based at least in part on the predetermined configuration.

In one aspect of the embodiment, a warming area provided by a first warming element overlaps with a warming area provided by a second warming element.

In one aspect of the embodiment, the body comprises at least two layers.

In one aspect of the embodiment, the blanket further comprises an insulating material situated between the at least two layers.

In one aspect of the embodiment, the body comprises a water resistant or waterproof material.

In one aspect of the embodiment, the body comprises a reflective material.

In one aspect of the embodiment, each of the one or more pockets comprises an elastic or elasticized material.

In one aspect of the embodiment, each of the one or more pockets comprises a material that controls the flow of air to a heating element inserted into the pocket.

In one aspect of the embodiment, the one or more pockets are arranged in a predetermined configuration such that overlapping regions of warmth are provided by the removable warming elements.

In another embodiment, a method for fabricating a warming blanket includes providing a body for the warming blanket. The method further includes providing one or more pockets removably connected to the body, wherein each of the one or more pockets is adapted to hold a respective removable heating element. In addition, the method can include removably mounting the one or more pockets to the body.

In yet another embodiment, a method of using a warming blanket can include providing a warming blanket with one or more pockets removably connected to a body, the pockets adapted to hold a respective removable heating element. In addition, the method can include inserting a respective heating element into each of the one or more pockets. Furthermore, the method can include positioning the warming blanket on a user.

In yet another embodiment, an easily opened, comfortable active warming device in the form of a sheet or blanket can be provided. The warming device can include a medical grade cloth that blends in with other operating room covers or drapes. It has a time course of at least 12 hours and is disposable once it cools. The warming device can be designed with a maximum temperature of 43°C and is actively warming within 20 minutes of opening. The warming device can include at least one application related to a medical procedure with an emphasis on providing pre, intra, and post operative warming for the clinical benefits as well as the patient satisfaction. The warming device can also find utility for non-medical use to prevent or otherwise minimize thermal discomfort in multiple settings.

In one aspect of the embodiment, the blanket can provide a maximum temperature of 43°C and lasts for approximately 12 hours or more.

In another aspect of the embodiment, the blanket can be a medical cloth, blue in color, and is 0.914m (3 feet) wide by 1.524m (5 feet) long.

In another aspect of the embodiment, the blanket can have white stitching on its border.

In another aspect of the embodiment, the blanket can come packaged in an airtight clear plastic 25.4cm (10 inch) by 43.18cm (17 inch) package with the energy sources already in place.

In another aspect of the embodiment, the blanket's packaging can be opened along a perforated easy open line, after which the blanket may be unfolded to its above size.

In another aspect of the embodiment, the blanket can include energy sources configured so that the exothermic reaction takes place to provide noticeable warmth to the patient within about 20 minutes of opening.

In yet another aspect of the embodiment, the continuation of warming can occur until a plateau is reached after one hour. This exothermic reaction can continue for at least 12 hours.

In another aspect of the embodiment, the weight of the blanket can be such that the blanket will lie atop of the person without slipping off.

In another aspect of the embodiment, the blanket can include three cutouts positioned to provide the ability to position the blanket either lengthwise (head to toe) on the patient or turning it 90° to allow exposure of abdomen and legs if the surgical procedure warrants this exposure.

In another aspect of the embodiment, the blanket can include symmetrical positioning of the energy sources.

In another aspect of the embodiment, the blanket can include asymmetrical positioning of the energy sources.

In yet another aspect of the embodiment, the blanket can include symmetrical position of the energy sources across the width by asymmetrical positioning of the energy sources across the length.

In another aspect of the embodiment, energy can be supplied via an exothermic chemical reaction that is initiated by exposure to air.

In another aspect of the embodiment, after activation of at least one heating element, the cover is sealed in a relatively air tight enclosure to inhibit the exothermic reaction of the at least one heating element.

One or more embodiments of the invention can provide certain aspects. It should be understood that the embodiments as claimed herein are not limited by the following recitation of potential aspects. It should be further understood that any particular embodiment as claimed might include one, or some combination of the recited aspects, but that any particular advantage should not be considered as necessarily present unless explicitly claimed or unless it necessarily follows from limitations that are explicitly claimed.

One aspect of certain embodiments is a self contained exothermic heating device that requires no attached external devices to provide the thermal input. An aspect of certain embodiments can provide warmth for up to about 12 hours supplying active warming for a thermal challenged person or patient. Another aspect of some embodiments of the invention is the disposability with decreased chance of infectious disease transfer.

Another aspect of certain embodiments is the lack of white noise produced that over time may have ill effects on medical staff in that environment. Another aspect of certain embodiments is the ability to lie relatively flat on a patient without any uplifting of the surgical drapes as can be caused by forced air warmers. In some instances, this has lead to surgical instrumentation falling from the sterile field. Yet another potential aspect is the ability to transport with the patient without requiring an attachment device, including traveling home with the patient.

Most facilities will utilize between 3 and 6 thick blankets per patient that presents for a procedure. This tremendous utilization of blankets results in large laundry bills for the facility that can eat into the profit margins. Certain embodiments of the invention can typically require only a single self-warming blanket. This could decrease the amount spent on laundry, as well as having an environmental effect by reducing water and energy utilization.

Other blankets, covers, apparatus, and processes according to various embodiments of the invention will become apparent with respect to the remainder of this document.

### BRIEF DESCRIPTION OF THE DRAWINGS

Reference will now be made to the accompanying drawings, which are not necessarily drawn to scale, and wherein:

FIG. 1 is a schematic diagram of one example warming blanket or cover according to embodiment of the invention.

FIG. 2 is a schematic diagram of another example warming blanket or cover according to embodiment of the invention.

FIG. 3 is a schematic diagram of yet another example warming blanket or cover according to embodiment of the invention.

FIG. 4 is a schematic diagram of one example pocket for a warming blanket or cover that may be utilized to secure a heat pad according to an embodiment of the invention.

FIG. 5 is a flowchart of one example method for fabricating a warming blanket or cover according to an embodiment of the invention.

FIG. 6 is a flowchart of one example method for using a warming blanket or cover according to an embodiment of the invention.

FIG. 7 is a top view of a folded blanket packaged in an airtight polymer according to an embodiment of the invention.

### DETAILED DESCRIPTION OF EMBODIMENTS OF THE INVENTION

Embodiments of the invention now will be described more fully hereinafter with reference to the accompanying drawings, in which embodiments of the invention are shown.. Like numbers refer to like elements throughout.

Embodiments of the invention may provide improved warming blankets, covers, and apparatus, and methods of fabricating and using the warming blankets, covers, and apparatus. Warming blankets, covers, and apparatus in accordance with various embodiments of the invention may include one or more pockets that facilitate the use of heat pads in association with the blankets, covers, or apparatus. Each of the one or more pockets may be configured to hold one or more heat pads. Each of the heat pads may be a heat pad that provides heat to at least a portion of a warming blanket or a heat zone of the warming blanket, cover, or apparatus. In this regard, multiple heat pads may be utilized in association with a warming blanket, cover, or apparatus, and the heat pads may be configured such that they have overlapping heat zones. Warming blankets, covers, and apparatus in accordance with certain embodiments of the invention can provide increased warmth, drapability, conformability as well as comfort over certain conventional blankets, covers, and apparatus in a variety of circumstances and applications.

Certain embodiments of the invention can eliminate or otherwise minimize the need to use a separate machine to provide thermal input for a patient. Such embodiments can be used to begin the pre-warming of a patient which may be shown to reduce the degree of cooling that the patient may experience during anesthesia. Furthermore, such embodiments can be used intraoperatively or as an adjunct to a forced air warmer and can provide significant thermal comfort and rewarming effect in the postoperative arena. In certain instances, such embodiments can be disposable.

As used herein, the terms "blanket", "cover", "apparatus", "warming device", and their respective pluralized forms can be used interchangeably, and are intended to describe a relatively flat structure that can be used to cover at least a portion of an object such as a person's or patient's body.

As used herein, the terms "heating element", "warming element", "heat pad", "energy source", "heating pad", "warming pad", "pad", "pod", and their respective pluralized forms can be used interchangeably, and are intended to describe an air-activated carbon, charcoal, or vermiculite heat pocket. In some embodiments, a different air-activated material and/or chemical composition can be utilized.

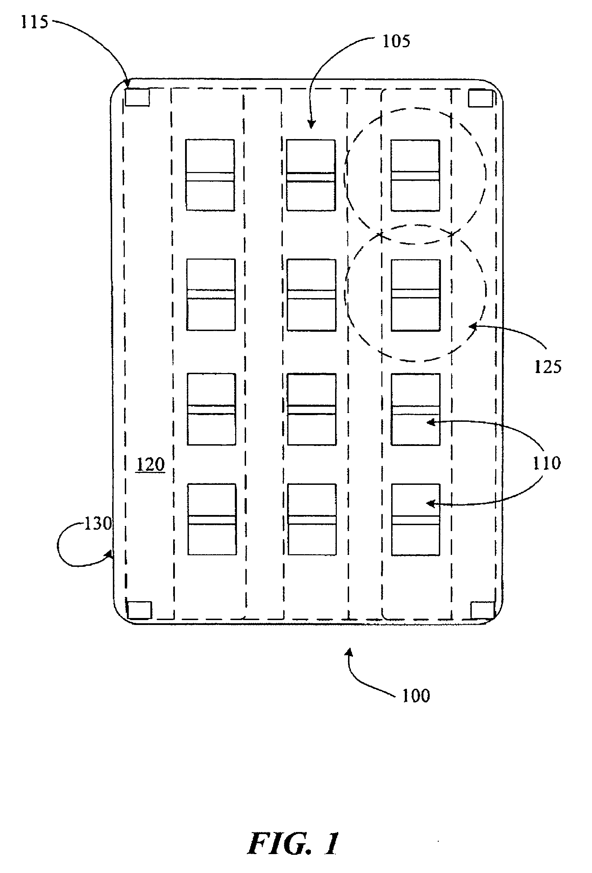

FIG. 1 is a schematic diagram of one example warming blanket 100 according to an embodiment of the invention. As shown in FIG. 1, the warming blanket 100 includes a body 105 and one or more pockets 110 removably connected to the body. Each of the one or more pockets 110 is removably attached to, removably affixed to, or otherwise removably mounted to the body 105. As explained in greater detail below, each of the one or more pockets 110 may be configured to hold one or more suitable heating elements or warming elements, such as one or more heat pads, heating pads, or warming pads.

A body 105 of a warming blanket 100 may have a wide variety of shapes and/or dimensions as desired in various embodiments of the invention. As shown in FIG. 1, the body 105 of the warming blanket 100 may have a substantially rectangular shape; however, in other embodiments of the invention, the body 105 may have a substantially square shape, substantially circular shape, substantially elliptical shape, a shape of a garment to be worn by a user or patient, or a shape that at least partially conforms to that of a user or patient, etc. Different shapes of warming blankets that are constructed from various materials may be designed to serve different functions. For example, a relatively large, substantially rectangular warming blanket may be constructed from relatively durable materials, and the warming blanket may be designed for outdoor and/or recreational uses. As another example, a relatively large, substantially rectangular blanket may be constructed from relatively lightweight, reflective materials such that the warming blanket can be easily packed or stored. In this regard, the warming blanket may be designed for use as an emergency blanket. As yet another example, a warming blanket may be formed with one or more cut-outs that conform to the shape of the human body, and the warming blanket may be designed for use in medical applications. In yet another embodiment, a relatively small size warming blanket with suitable cut-outs and/or material construction can be designed for use by an infant or child. In another embodiment, a warming blanket can be shaped to cover a person's torso in the form of a warming jacket. Other examples of warming blanket constructions and/or designs according to embodiments of the invention will be apparent.

According to an aspect of the invention, the body 105 may include a first surface 120 and a second surface 130. The first surface 120 of the body 105 may also be referred to as the internal surface of the warming blanket 100 or the back side of the warming blanket 100. Likewise, the back surface 130 of the body 105 may also be referred to as the external surface of the warming blanket 100 or the face side of the warming blanket 100. In certain embodiments of the invention, the second surface 130 may have a substantially smooth and/or have a substantially uniform surface. In use, the first surface 120 of the body 105 may be in direct contact with a user of the warming blanket 100. As shown in FIG. 1, one or more pockets 110 configured to hold respective heating or warming elements are removably connected to the first surface 120. In this regard, when the warming blanket 100 is in use, the heating or warming elements may provide additional heat to the user of the warming blanket 100. One suitable example of a heating or warming pad can be an air activated, carbon or charcoal powder filled pouch. In other embodiments, other types of individual heating or warming pads or air-activated materials or chemical compositions can be used.

In accordance with various embodiments of the invention, the warming blanket 100 may be constructed, manufactured, fabricated, and/or formed from a wide variety of different types of materials, different weights of materials, and/or combinations of different types and weights of materials. Additionally, as desired in various embodiments, the first surface 120 and the second surface 130 of the warming blanket 100 may be formed from different materials. For example, in one embodiment, the first surface 120 may be formed from one or more polypropylene (PP) materials, spunbond (SB) materials, spunbond/melt blown/spunbond (SMS) materials, spunbond/melt blown/melt blown/spunbond (SMMS) materials, low density polyethylene (LDPE) materials, cotton, rayon, synthetics, one or more breathable materials, one or more non-woven materials, one or more natural materials, one or more recycled or reworked materials, and/or one or more manmade or artificial materials. The second surface 130 may be formed from one or more polypropylene (PP) materials, spunbond (SB) materials, polypropylene spunbond (PPSB) materials, low density polyethylene (LDPE) materials, cotton, rayon, synthetics, one or more non-woven materials, one or more natural materials, and/or one or more manmade or artificial materials.

In one embodiment, certain weights of specific materials can be used for a warming blanket shown in FIG. 1. For example, a warming blanket can comprise a first surface of a white coated PPSB material with a weight of approximately 35 g/m2, and can further comprise a second surface of a dark blue LDPE material with a weight of approximately 30 g/m2. Various other embodiments can utilize different weights of similar or different materials depending at least in part on the desired comfort, drapability, conformability and/or warmth properties to be achieved.

Alternatively, the body 105 of the warming blanket 100 may be formed as a single component, and the first surface 120 and the second surface 130 may be formed utilizing the same or otherwise compatible materials and/or combinations of materials. Additionally, in certain embodiments, the first surface 120 and/or the second surface 130 may be constructed or formed to include a plurality of similar or different layers of material.

In any instance, the combination of materials selected for a warming blanket in accordance with certain embodiments of the invention can be based at least in part on the desired comfort, drapability, conformability and/or warmth for the warming blanket. For example, one or more predefined standards of comfort drapability, conformability and/or warmth may be accounted for in selecting the combination of materials for a warming blanket in accordance with an embodiment of the invention.

Furthermore, in other warming blanket embodiments, certain desired characteristics such as moisture permeability, oxygen transfer rates (OTR), and moisture vapor transmission rates (MVTR) can be accounted for and engineered in a warming blanket by selecting one or more suitable materials for assembly to achieve any desired combined characteristics for the assembled warming blanket.

According to one embodiment of the invention, the first surface 120 and/or the second surface 130 may be formed from one or more water resistant and/or waterproof materials. In another embodiment, the first surface 120 and/or the second surface 130 may include one or more layers of water resistant and/or waterproof material. In another embodiment, the first surface 120 and/or the second surface 130 may be coated with or otherwise treated with a water repellant.

In other embodiments of the invention, the first surface 120 and/or the second surface 130 may include a stain resistant layer and/or be treated with a stain resistant chemical or treatment. In yet other embodiments, the first surface 120 and/or the second surface 130 may include a reflective material or be treated to be reflective with a chemical, treatment, or layer. In this regard, the visibility of the blanket 100 may be enhanced.

In certain embodiments of the invention, the body 105 of the blanket 100 may include a single component with both a first surface 120 and a second surface 130. In other embodiments of the invention, the body 105 may include a plurality of components. For example, the first surface 120 and the second surface 130 of the body 105 may be separate components of the blanket 100. In embodiments where the body 105 is formed from a plurality of components, the components may be fixedly or removably connected and/or attached to one another via any suitable connectors, connecting techniques, and/or connection means. For example, the first surface 120 and the second surface 130 of the body 105 may be connected to one another via sewing, stitching, bonding, application of an adhesive, threads, ties, use of snaps, use of ultrasonic welding, use of velcro, use of one or more zippers, etc.

Additionally, in various embodiments of the invention, one or more insulation layers and/or insulating materials may be situated between the first surface 120 and the second surface 130. A wide variety of different insulating materials may be utilized as desired in various embodiments of the invention, such as, natural insulating materials (e.g., goose down), artificial insulating materials (e.g., Thermacore™ insulation) or reflective type materials (e.g. aluminum or tin reflective foil).

In accordance with one embodiment of the invention, the warming blanket 100 comprises a body and one or more pockets removably connectable to the body; the blanket may include one or more weights 115 that may assist in holding the warming blanket 100 in place or with respect to a user or patient. For example, if the warming blanket is utilized in a medical or post-operative environment, the one or more weights 115 may assist in holding the warming blanket 100 down over a patient's body and minimizing the movement of the warming blanket 100 that is caused by movement of the patient or attending medical personnel. In another example, if the warming blanket is utilized in an outdoor environment (e.g., sporting event, picnic, etc.), the one or more weights 115 may assist in holding the warming blanket 100 down and minimizing the movement of the warming blanket 100 that is caused by external forces, such as, the wind. The one or more weights 115 may be situated at various locations on the warming blanket 100 as desired in various embodiments of the invention, such as, at one or more corners or along one or more edges of the warming blanket 100. As shown in FIG. 1, the a weight 115 may be situated at each of the four corners of the warming blanket 100. Each of the one or more weights 115 may be permanently or removably affixed, attached, or otherwise mounted to the first surface 120 and/or the second surface 130 of the warming blanket by any suitable connectors, connecting techniques, and/or connection means. For example, each of the weights may be connected to the warming blanket 100 via sewing stitching, bonding, application of an adhesive, threads, ties, use of snaps, etc.

As shown in FIG. 1, one or more pockets 110 are removably attached to the first surface 120 of the warming blanket 100. Although the warming blanket 100 is described as having pockets 110 removably attached to the first surface 120 or internal surface of the body 105, various embodiments of the invention may include one or more pockets 110 that are removably attached to, removably mounted to, or removably affixed to the second surface 130 of the body 105. Additionally, in certain embodiments, one or more pockets 110 may be removably attached to the first surface 120 and/or the second surface 130 so that the one or more pockets 110 are situated between the first surface 120 and the second surface 130 (i.e., within the blanket). For example, in an embodiment where the first surface 120 and the second surface 130 are removably connected (e.g., via a zipper, etc.), the one or more pockets 110 may be removably attached on removably mounted to the first surface 120 or the second surface 130 of the blanket, and between the first surface 120 and second surface 130 of the warming blanket 100.

According to an aspect of the invention, one or more warming elements or heating elements may be situated in or placed in each of the one or more pockets 110, the said pockets being removably connectable to the body of the blanket. A wide variety of different warming or heating elements may be utilized as desired in various embodiments of the invention, such as, warming pads or heat pads. Additionally, a wide variety of different warming pads may be utilized as desired in various embodiments of the invention. According to certain embodiments of the invention, each of the warming elements may be an air activated charcoal and/or an air activated vermiculite warming pad. Each of the warming pads may be a disposable warming pad that contains a combination of materials such as iron powder, water, salt, activated charcoal, and/or vermiculite. When a warming pad is exposed to oxygen or otherwise activated, a chemical reaction may be initiated within the warming pad that causes heat to be emitted by the pad. The warming pad may continue to emit heat at a predetermined temperature, for example, about 48.888 degrees Celsius (120 degrees Fahrenheit) to about 60 degrees Celsius (140 degrees Fahrenheit) for a predetermined duration of time, for example, about 2 to about 30 hours. Furthermore, each warming pad may be configured so that an associated exothermic reaction takes place to provide noticeable warmth within about 20 minutes of activation.

Each of the one or more warming pads may be removably situated within or inserted into a pocket 110 of the warming blanket 100; the pocket being removably connectable to the body of the blanket. When activated and situated within a pocket 110, a warming pad may emit heat that supplements any heat or warmth provided by or otherwise retained by the materials of the warming blanket 100. As shown in FIG. 1, each of the activated warming pads may provide heat or warmth to a certain area of the warming blanket 100. In other words, each of the activated warming pads may emit heat that affects the blanket 100 within a predefined heat zone 125 for the warming pad. Each of the predefined heat zones 125 may affect a substantially circular or other geometrically-shaped area of the warming blanket 100 that extends outwardly from the heat pad. Additionally, as shown in FIG. 1, various predefined heat zones 125 may overlap one another. In this regard, a plurality of warming pads may be utilized to provide heat or warmth to selected portions of a warming blanket or to all or substantially all of the warming blanket. In this manner, one or more overlapping predefined heat zones may provide additional heating or otherwise prolonged heating to certain or otherwise preselected areas of a user's body when the blanket 100 is worn by or placed over a user.

Although the embodiments of the invention discussed herein are described as utilizing warming elements, such as, warming pads, in certain embodiments of the invention, cooling elements, such as cold packs, may be inserted as desired into the pockets 110 of the blanket 100 according to another embodiment of the invention. Each of the cooling elements may produce a predetermined temperature for a predetermined duration of time, or otherwise form one or more cooling zones for the blanket 100. The cooling zones can be defined as geometrically-shaped areas similar in shape and orientation as the predefined heat zones 125 shown in FIG. 1.

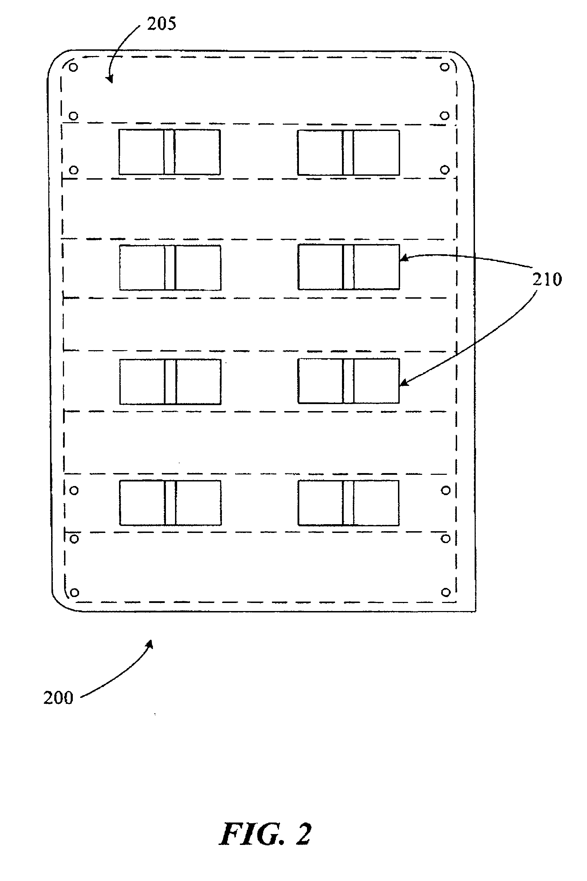

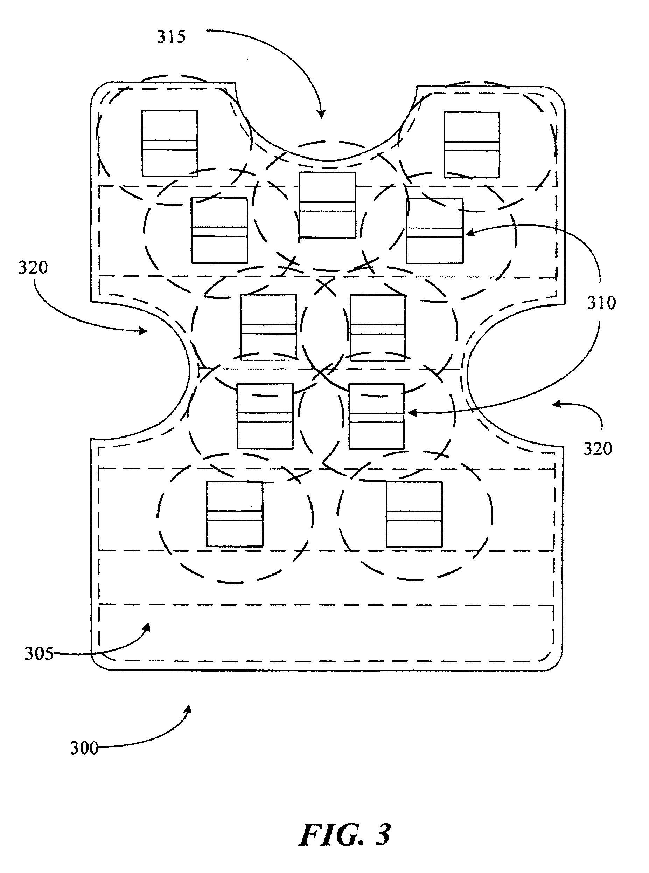

According to various embodiments of the invention, the one or more pockets 110 may be situated in a wide variety of different configurations as desired. In this regard, the predefined heat zones 125 emitted or generated by heat pads that are placed within the one or more pockets 110 may be configured in a wide variety of different ways as desired. A few examples of various configurations of the one or more pockets are illustrated in FIGS. 1-3. For example, in FIG. 1, four rows of three pockets each are utilized. As another example, in FIG. 2, four rows of two pockets each are utilized. As yet another example, in FIG. 3, eleven similar sized pockets may be situated in a specific geometric or non-geometric-shaped configuration to apply heat or cooling to predetermined areas of a user's body when heating or warming elements are mounted within the pockets and the blanket is worn by or placed over a user.

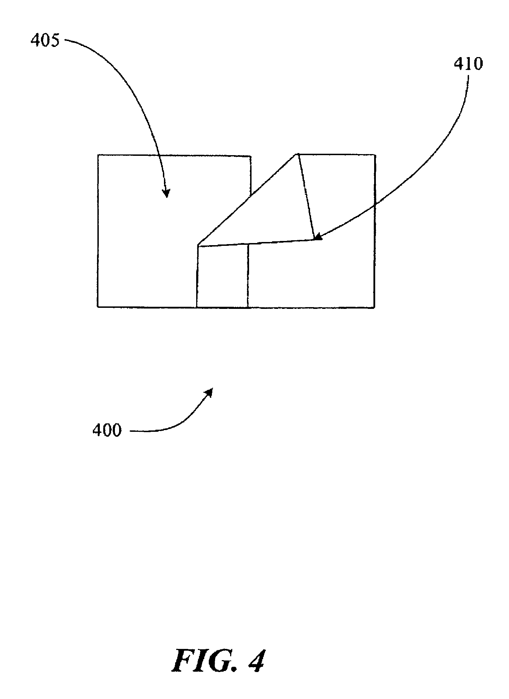

According to an aspect of the invention, each of the one or more pockets 110 is configured to hold at least one warming or heating element, such as a warming pad. The one or more pockets being removably connectable to the body. Additionally, in embodiments of the invention where the one or more pockets are situated such that they may apply heat or cooling to predetermined areas of a user's body when the blanket 100 is worn by a user, each of the one or more pockets 110 may be designed to hold a warming pad such that a warming surface of the warming pad is substantially close to a surface of the pocket 110. According to certain embodiments of the invention, each of the one or more pockets 110 may have a design that is similar to that of a pillow sham, although other designs may be utilized as desired. The construction of one example pocket, such as pocket 110, is illustrated in FIG. 4 and described in the associated text below.

In certain embodiments of the invention, each of the one or more pockets 110 may have a shape that generally corresponds to the warming pads that are intended to be inserted into or situated with the pockets 110. As shown in FIG. 1, each of the one or more pockets may have a substantially rectangular shape; however, in other embodiments of the invention, each of the one or more pockets 110 may have a substantially square shape, a substantial circular shape, a substantially elliptical shape, etc. With continued reference to FIG. 1, each of the one or more substantially rectangular pockets 110 are situated such that the length dimension of the pockets 110 is substantially parallel to the length dimension of the warming blanket 100. However, in various embodiments of the invention, each of the pockets 110 may be situated as desired in a wide variety of different directions with respect to the blanket 100. For example, as shown in FIG. 2, each of the one or more pockets 110 may be situated such that the length dimension of the pockets 110 is substantially parallel to the width dimension of the warming blanket 100.

Each of the one or more pockets 110 may be constructed and/or formed from a wide variety of different materials or combinations of materials as desired in various embodiments of the invention. For example, each of the one or more pockets may be constructed from similar materials to those utilized to form or construct the first surface 120 of the warming blanket 100. In various embodiments of the invention, a pocket 110 may be formed from one or more polypropylene (PP) materials, spunbond (SB) materials, spunbond/melt blown/spunbond (SMS) materials, spunbond/melt blown/melt blown/spunbond (SMMS) materials, low density polyethylene (LDPE) materials, cotton, rayon, synthetics, one or more non-woven materials, one or more breathable materials, one or more natural materials, one or more recycled or reworked materials, and/or one or more manmade or artificial materials.

In one embodiment, certain weights of specific materials can be used for one or more pockets associated with a warming blanket shown in FIG. 1. For example, at least one pocket can comprise a nontreatment SMMS material with a weight of approximately 50 g/m2. Various other embodiments can utilize different weights of similar or different materials depending at least in part on the desired comfort, drapability, conformability and/or warmth properties to be achieved.

According to an aspect of the invention, one or more pockets are removably connectable to the body of the blanket wherein each of the one or more pockets 110 may incorporate an elasticized or elastic material. The material may be stretched in order to place a warming pad within the pocket 110, and the material may then substantially return to its original position to hold the inserted heat pad in place. In this regard, an inserted warming pad may be prevented from shifting or moving within the pocket 110. Additionally, the elasticized material of the pocket 110 may exert a force on the warming pad that facilitates holding the warming pad and its contents in place. In this regard, the shifting of the internal ingredients of a warming pad (e.g., iron powder, salt, charcoal, etc.) may be reduced.

Additionally, in various embodiments of the invention, the one or more pockets 110 may be constructed from one or more materials that facilitate the control of airflow to warming pads inserted into the blanket 100. In this regard, the one or more pockets 110 may facilitate at least partial control over the duration of time in which inserted warming pads remain active. For example, by limiting and/or inhibiting the flow of air to a warming pad, the amount of oxygen provided to the warming pad may be limited, thereby facilitating a longer or shorter activation period for heat pad.

According to an aspect of the invention, one or more warming pads may be removably inserted into a pocket 110 and removed as needed. The one or more pockets being removably connectable to the body. The one or more warming pads inserted into each respective pocket 110 may be replaced once their fuel source has been expended or the desired temperature from the respective pocket 110 needs to be modified. In this regard, the warming blanket 100 may be a reusable warming blanket 100 in which warming pads may be used and replaced prior to subsequent uses.

Each of the one or more pockets 110 is removably connected and/or removably attached to the body 105 of the warming blanket 100 via any suitable connectors, connecting techniques, and/or connection means. For example, a pocket 110 may be connected to the body 105 of the warming blanket 100 via ties, use of snaps, use of velcro, use of one or more zippers, etc.

In one embodiment, certain weights of specific materials can be used for ties and/or threads associated with a warming blanket in FIG. 1. For example, at least one tie can comprise a white PPSB material with a weight of approximately 40 g/m2, and at least one thread can comprise a white spun 60/2 material. Various other embodiments can utilize different weights of similar or different materials depending at least in part on the desired comfort, drapability, conformability and/or warmth properties to be achieved.

FIG. 2 is a schematic diagram of another example warming blanket 200 in accordance with an illustrative embodiment of the invention. The components of the warming blanket illustrated in FIG. 2 may be substantially similar to those of the warming blanket 100 illustrated in FIG. 1; however, the configuration of the pockets may be different. As shown in FIG. 2, the warming blanket 200 has a body 205 with a plurality of pockets 210 removably connected to the body 205. The warming blanket 200 of FIG. 2 includes a fewer number of pockets 210 than those illustrated for the warming blanket 100 of in FIG. 1. The use of a fewer number of pockets 210, along with fewer materials used to construct the warming blanket 200, may lead to a warming blanket 200 that has a relatively lower overall weight. If a fewer number of warming pads are inserted into a warming blanket 200, then a warming blanket in use will have a relatively lower weight. Additionally, the use of a fewer number of warming pads may lessen the warmth or heat provided by the warming blanket 200. In certain embodiments, a warming blanket can include a particular number of warming pads configured so that the associated exothermic reaction with each respective pad takes place to provide noticeable warmth within about 10 to 20 minutes of activation. Accordingly, during the design of a warming blanket, the size of the warming blanket, desired heat region to be provided by the warming pads, and/or the weight of the warming blanket may be taken into account.

In one embodiment, certain weights of specific materials can be used for a warming blanket shown in FIG. 2. For example, a warming blanket can comprise a first surface of a PPSB material with a weight of approximately 40 g/m2, and can further comprise a second surface of a coated blue #Y542U LDPE material with a weight of approximately 20 g/m2. Various other embodiments can utilize different weights of similar or different materials depending at least in part on the comfort, drapability, conformability, and/or warmth properties to be achieved.

In one embodiment, certain weights of specific materials can be used for one or more pockets associated with a warming blanket shown in FIG. 2. For example, at least one pocket can comprise a nontreatment white #15 SMMS material with a weight of approximately 50 g/m2. Various other embodiments can utilize different weights of similar or different materials depending at least in part on the comfort, drapability, conformability and/or warmth properties to be achieved.

In one embodiment, certain weights of specific materials can be used for ties and/or threads associated with a warming blanket in FIG. 2. For example, at least one tie can comprise a white PPSB material with a weight of approximately 38 g/m2, and at least one thread can comprise a white spun 60/2 material. Various other embodiments can utilize different weights of similar or different materials depending at least in part on the comfort, drapability, conformability and/or warmth properties to be achieved.

With continued reference to FIG. 2, the warming blanket 200 illustrated may be utilized, for example, as an emergency blanket. The incorporation of fewer pockets 210 may lead to a less bulky and relatively lower weight blanket that may be packed or stowed in an emergency kit, hiking pack, vehicle, etc. Additionally, the warming blanket 200 may be constructed from relatively light weight reflective materials, as described above.

Additionally, the warming blanket 200 may include one or more components that facilitate the folding, packing, and/or storage of the warming blanket 200. For example, one or more snaps, zippers, buttons, etc. may be positioned on the warming blanket 200 to facilitate maintaining the warming blanket 200 in a folded and/or packable configuration. In one embodiment, the warming blanket 200 may include one or more snaps situated at one or more corners of the warming blanket 200, and the one or more snaps may be utilized to maintain the warming blanket 200 in a folded position. In another embodiment, the warming blanket 200 may include a storage portion or pocket portion, and the warming blanket 200 may be inserted into or packed into the storage portion. In this regard, the warming blanket 200 may be packed and/or stored in a relatively portable configuration.

FIG. 3 is a schematic diagram of yet another example warming blanket 300 in accordance with an illustrative embodiment of the invention. The components of the warming blanket illustrated in FIG. 3 are substantially similar to those of the warming blanket 100 illustrated in FIG. 1. However, as with FIG. 2, the configuration of the pockets may be different. As shown in FIG. 3, the warming blanket 300 includes a body 305 with a plurality of pockets 310 removably connected to the body 305. The plurality of pockets 310 may be arranged in a configuration that provides a desired warming area or a desired group of heat zones for the warming blanket 300. Additionally, in accordance with various embodiments of the invention, the warming blanket 300 illustrated in FIG. 3 may be constructed from one or more materials suitable for use in medical and/or surgical applications. For example, the warming blanket 300 may be constructed or formed from one or more polypropylene (PP) materials, spunbond (SB) materials, spunbond/melt blown/spunbond (SMS) materials, spunbond/melt blown/melt blown/spunbond (SMMS) materials, low density polyethylene (LDPE) materials, cotton, rayon, synthetics, one or more non-woven materials, one or more breathable materials, one or more natural materials, one or more recycled or reworked materials, and/or one or more manmade or artificial materials.

In accordance with various embodiments of the invention, the plurality of pockets 310 may be configured to provide warmth, or heat to a patient in association with various medical and/or surgical procedures, such as, pre-operative, intra-operative and/or post-operative procedures. The plurality of pockets 310 may be situated in such a manner that relatively optimal heating or warming is provided to a patient. Additionally, the plurality of pockets 310 may be situated such that overlapping regions or areas of warmth are provided by associated warming pads. The configuration and/or locations of the pockets 310 and their associated overlapping regions of warmth may be predetermined in order to provide relatively optimal heating to a patient. Furthermore, a warming blanket can include a certain number of warming pads configured so that the associated exothermic reaction with each respective pad takes place to provide noticeable warmth within about 10 to 20 minutes of activation.

In one embodiment, certain weights of specific materials can be used for one or more pockets associated with a warming blanket shown in FIG. 3. For example, at least one pocket can comprise a nontreatment blue #15 SMMS material with a weight of approximately 52 g/m2. Various other embodiments can utilize different weights of similar or different materials depending at least in part on the comfort, drapability, conformability and/or warmth properties to be achieved.

In one embodiment, certain weights of specific materials can be used for ties and/or threads associated with a warming blanket in FIG. 3. For example, at least one tie can comprise a white PPSB material with a weight of approximately 40 g/m2, and at least one thread can comprise a white spun 60/2 material and/or a spun Venus white material. Various other embodiments can utilize different weights of similar or different materials depending at least in part on the comfort, drapability, conformability and/or warmth properties to be achieved.

Additionally, the body 305 of the warming blanket 300 may include one or more cut-out portions 315, 320 that are associated with the head and/or limbs of a patient or user of the warming blanket 300. As shown in FIG. 3, a cut-out 315 may be provided for a patient's head and two respective cut-outs 320 may be provided for a patient's arms. By providing these cut-outs, the warming blanket 300 will facilitate accommodating protruding portions of a patient's body. In this regard, a desired warming of certain portions of a patient's body may be achieved in a medical-type or other application. Additionally, the cut-outs can facilitate greater flexibility in the movement of the head and/or limbs of the patient while maintaining the application of heat to certain other portions of a patient's body.

Warming blankets in accordance with various embodiments of the invention may be utilized in a wide variety of different applications and/or for a wide variety of different purposes. Some examples of applications in which the warming blankets may be utilized are outdoor activities, recreational activities, sporting activities, travel activities, emergency situations, and or medical applications. Additionally, the warming blankets may be utilized to provide a wide variety of different benefits to a user, such as, comfort, warmth, protection from the elements, stress relief, muscle tension relief, therapeutic purposes, aid in circulation, arthritis relief, etc.

As discussed above, the combination of materials selected for a warming blanket in accordance with certain embodiments of the invention can be based at least in part on comfort drapability, conformability and/or warmth. For example, one or more predefined standards of comfort, drapability, conformability and/or warmth may be accounted for in selecting the combination of materials for a warming blanket in accordance with an embodiment of the invention.

Furthermore, in other warming blanket embodiments, certain characteristics such as moisture permeability, oxygen transfer rates (OTR), and moisture vapor transmission rates (MVTR) can be accounted for and engineered in a warming blanket by selecting one or more suitable materials for assembly to achieve any desired combined characteristic for the assembled warming blanket.

FIG. 4 is a schematic diagram of one example pocket 400 that may be utilized to secure a warming pad in accordance with various embodiments of the invention. As shown in FIG. 4, the pocket 400 may include a first portion 405 and a second portion 410. The first portion 405 may extend from one edge of the pocket 400 and the second portion 410 may extend from an opposite edge of the pocket 400. In certain embodiments of the invention, the first portion 405 and the second portion 410 may overlap one another. The amount of overlap may vary as desired in different embodiments of the invention. For example, in one embodiment, the amount of overlap may be approximately 2.0 cm (or less than about one inch). In alternate embodiments of the invention, there may be no overlap between the first portion 405 and the second portion 410.

By providing a first portion 405 and a second portion 410 of a pocket 400, the pocket 400 may facilitate the insertion of a warming pad, and the pocket may maintain or hold an inserted warming pad in place. For example, a warming pad may be inserted between the first portion 405 and the second portion 410 of the pocket 400, and the first portion 405 and/or the second portion 410 may be adjusted or otherwise manipulated such that the warming pad is situated beneath both the first portion 405 and the second portion 410 of the pocket 400. In this regard, a warming pad may be removably secured within the pocket 400.

According to an aspect of the invention, the first portion 405 and/or the second portion 410 may include and/or be constructed at least in part from one or more stretchable, elasticized or elastic materials. In this regard, the first portion 405 and/or the second portion 410 may be flexible, thereby permitting the insertion of one or more warming pads. Additionally, the first portion 405 and/or the second portion 410 may supply a compressive force to an inserted heat pad that facilitates holding the warming pad and/or its internal components in place.

In addition to the first portion 405 and the second portion 410 of the pocket 400, which may form an outer surface of the pocket 400, the pocket 400 may include an internal surface (not shown). The internal surface may be configured to contact the body of a warming blanket in which the pocket 400 is utilized; the pocket being removably connectable to the body. Additionally, the internal surface may provide additional support to a warming pad or other warming element that is inserted into the pocket 400.

The pocket 400 illustrated in FIG. 4 is only one example of a pocket that may be utilized in various embodiments of the invention. A wide variety of different types of pockets may be utilized as desired in various embodiments of the invention. For example, a pocket may be utilized that includes only a single sleeve portion that facilitates the removable insertion of a warming pad. The sleeve portion may be open on at least one side to facilitate the insertion of a warming pad. Additionally, in certain embodiments, an open side of the pocket may include a stretchable, elastic, or elasticized material that facilitates holding an inserted warming pad in place. In other embodiments, the open side of a pocket may include one or more snaps, zippers, and/or other fastening devices that may be utilized to close the open side of the pocket and hold an inserted warming pad in place.

Pockets in accordance with various embodiments of the invention may include any number of components or pieces, such as, one piece, two pieces, three pieces, etc. For example, in certain embodiments, a pocket may include a single piece of stretchable or elastic material that facilitates holding an inserted warming pad in place. For example, the pocket may include a single piece of elastic material that may conform to the shape of an inserted warming pad and facilitate holding the inserted warming pad in place.

According to one aspect of the invention, a pocket may facilitate a relatively easy insertion and removal of a warming pad into and out of the pocket wherein the pocket is removably connectable to the body. Additionally, the pocket may facilitate the retention of the warming pad in place once the warming pad is inserted.

Additionally, in certain embodiments of the invention, a pocket may be formed or constructed from one or more materials that are suitable for medical applications, such as, polypropylene (PP) materials, spunbond (SB) materials, spunbond/melt blown/spunbond (SMS) materials, spunbond/melt blown/melt blown/spunbond (SMMS) materials, low density polyethylene (LDPE) materials, cotton, rayon, synthetics, one or more non-woven materials, one or more breathable materials, one or more natural materials, one or more recycled or reworked materials, and/or one or more manmade or artificial materials. The materials utilized in the construction of the pocket may be relatively sterile materials that facilitate cleanliness within a medical environment.

Additionally, in certain embodiments of the invention, a pocket may be formed without fastenings, such as zippers. The absence of fastenings may minimize other objects snagging or catching the blanket, and may further facilitate the ease and flexibility of inserting and removing warmers from the pockets. Additionally, the absence of fastenings may provide a pocket with a relatively higher sterility and/or cleanliness as compared to a pocket that includes one or more fastenings.

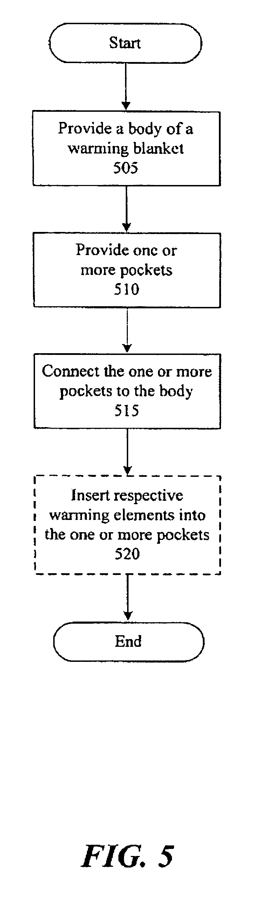

FIG. 5 is a flowchart of one example method 500 for fabricating a warming blanket in accordance with various embodiments of the invention. The method 500 may begin at block 505.

At block 505, a body of a warming blanket is provided. The body may be formed from a wide variety of different materials and/or combinations of materials as desired in various embodiments of the invention, as discussed in greater detail above with reference to FIGs. 1 - 3.

At block 510, one or more pockets is provided. Each of the one or more pockets is configured to or adapted to have a respective warming element, such as, a warming pad, removably inserted to rest within the pocket. Each of the one or more pockets may be formed from a wide variety of different materials and/or combinations of materials as desired in various embodiments of the invention, as discussed in greater detail above with reference to FIGs. 1 - 4.

At block 515, the one or more pockets is removably connected or otherwise removably mounted to the body via any number of suitable connections, connection techniques, and/or connection means, as discussed in greater detail above with reference to FIGs. 1 - 4. The one or more pockets is removably connected or removably mounted to the body in a wide variety of different configurations that may determine at least in part the heating area that is provided by the warming blanket once one or more heat pads are inserted.

Once the one or more pockets have been removably connected or removably mounted to the body at block 515, operations may continue at block 520, which may be optional in certain embodiments of the invention. At block 520, respective warming elements, such as, heat pads, may be inserted into one or more of the pockets that have been removably connected to the body of the blanket.

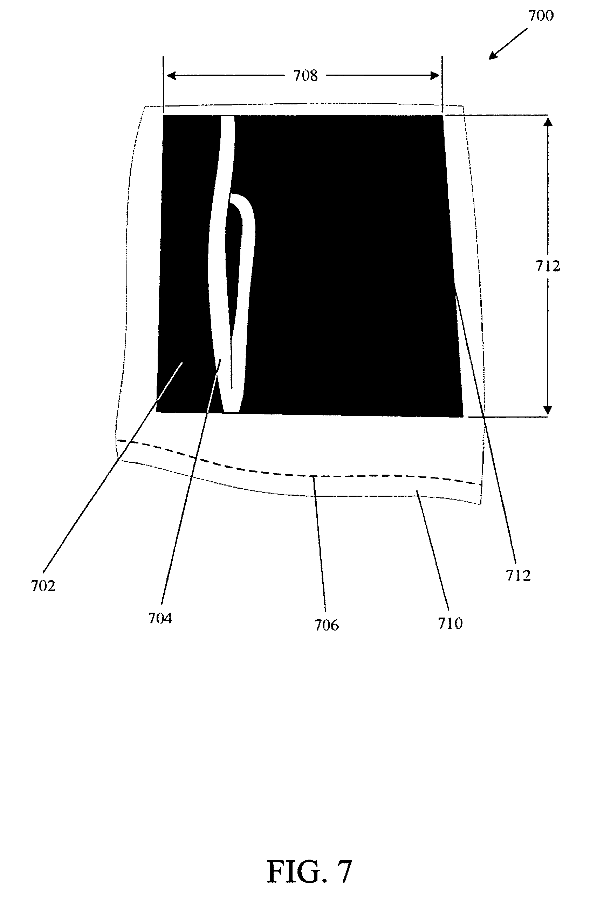

Once constructed, a warming blanket may be situated in a suitable package or within a suitable packaging material for distribution. For example, a constructed blanket may be placed in an air-tight sealed package for distribution as a ready-to-use blanket, an example of which is described and shown in FIG. 7 below.

The method 500 may end following block 520.

The operations described in the method 500 of FIG. 5 do not necessarily have to be performed in the order set forth in FIG. 5, but instead may be performed in any suitable order. Additionally, in certain embodiments of the invention, more or less than all of the operations set forth in FIG. 5 may be performed.

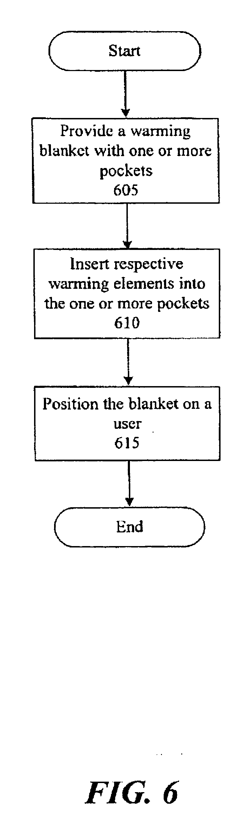

FIG. 6 is a flowchart of one example method 600 for using a warming blanket in accordance with various embodiments of the invention. The method 600 may begin at block 605.

At block 605, a warming blanket is provided. The warming blanket includes one or more pockets that are configured or adapted to have a respective warming element, such as, a warming pad, inserted to rest within the pocket. The one or more pockets being removably connected to the body of the blanket. Additionally, the pockets may be arranged in a predetermined configuration on the warming blanket to provide predefined heat zones or overlapping predefined heat zones. In some embodiments, the warming blanket may also include one or more cut-out portions as described above with reference to FIG. 3.

At block 610, respective warming elements, such as warming pads, may be removably inserted into one or more of the pockets of the provided warming blanket. Each of the respective warming elements may be activated either before or after they are inserted into a pocket.

At block 615, the warming blanket may be positioned on one or more users of the blanket. For example, in a medical application, the warming blanket may be positioned with respect to or mounted directly on or with respect to a portion of a user or patient's body. In this manner, the warming blanket can provide predefined heat zones or overlapping predefined heat zones to a user or patient's body.

In one aspect of an embodiment of the invention, the blanket can be positioned adjacent to the patient in a pre-operative room.

In one aspect of an embodiment of the invention, the blanket can be positioned adjacent to the patient in a post-operative room.

In one aspect of an embodiment of the invention, the blanket can be exposed to air approximately 20 minutes before positioning the cover adjacent to the patient.

Many modifications and other embodiments of the inventions set forth herein will come to mind to one skilled in the art to which these inventions pertain having the benefit of the teachings presented in the foregoing descriptions and the associated drawings. Therefore, it is to be understood that the inventions are not to be limited to the specific embodiments disclosed and that modifications and other embodiments are intended to be included within the scope of the appended claims. Although specific terms are employed herein, they are used in a generic and descriptive sense only and not for purposes of limitation.

## Claims

1. A method of using a warming blanket (100, 200, 300), the method comprising the steps of:
providing a warming blanket with a body (105, 205, 305) and one or more pockets (110, 210, 310) removably connected to the body, each of the one or more pockets comprising a heating element;
and,
positioning the warming blanket on a user;
**characterised by** the step of:
removably inserting the said heating element into one or more of the said pockets (110, 210, 310) such that the heating element may be replaced.

2. The method of claim 1, further comprising exposing the warming blanket (100, 200, 300) to air.

3. The method of claims 1 or 2 wherein the positions of the said pockets (110, 210, 310) provide for heat distribution that provides suitable thermal comfort to the user yet simultaneously allows for surgical access to the user.

4. The method of any preceding claim, wherein the warming blanket (110, 210, 310) is positioned adjacent to the user in either a pre-operative room or a post-operative room.

5. The method of any preceding claim, wherein the warming blanket (110, 210, 310) is exposed to air approximately twenty minutes before positioning the warming blanket adjacent to the user.

6. The method of any preceding claim, wherein the warming blanket (110, 210, 310) is self-warmed by an exothermic reaction in the chemical composition in each heating element, and wherein the exothermic reaction lasts approximately twelve hours.

7. The method of any preceding claim, wherein after activation of at least one heating element, the warming blanket (110, 210, 310) is sealed in a relatively air tight enclosure to inhibit the exothermic reaction of the at least one heating element.

8. A warming blanket (110, 210, 310), comprising:
a body (105, 205, 305); and
one or more pockets (110, 210, 310) removably connectable to the body, wherein each of the one or more pockets is adapted to hold a respective heating element;
**characterised in that**:
one or more of the pockets (110, 210, 310) are adapted so that the said heating element is removably insertable into the pocket, wherein:
A) the pocket comprises a single sleeve portion that facilitates the removable insertion of the heating element; or,
B) the pocket comprises an elasticized or elastic material that may be stretched in order to place the heating element within the pocket; or,
C) the pocket comprises a first portion (405) and a second portion (410) wherein:
i) the heating element is insertable between the first portion (405) and the second portion (410); or,
ii) the first portion (405) and/or the second portion (410) are flexible, thereby permitting the insertion of the heating element.

9. The warming blanket (100, 200, 300) of claim 8 comprising warming elements, wherein each of the respective warming elements comprises at least one of the following: carbon, charcoal, vermiculite, or an air-activated chemical composition, a chemical composition having an exothermic reaction when activated by exposure to air, a sufficient amount and distribution of the chemical composition so that the exothermic reaction lasts approximately twelve hours after exposure to air, an exothermic reaction beginning approximately twenty minutes after exposure to air, or a reaction time to provide warmth approximately a time of user pre-procedure intake admission.

10. The warming blanket (100, 200, 300) of claims 8 or 9, wherein the body (105, 205, 305) comprises any one of the following: a substantially rectangular shape, a substantially square shape, a substantially elliptical shape, a substantially circular shape, a garment shape, a shape that substantially conforms to the shape of a human body; one or more cut-out portions to accommodate one or more respective appendages of the human body; a water resistant or waterproof material; or at least one reflective material.

11. The warming blanket (100, 200, 300) of any of claims 8 to 10, wherein the body (105, 205, 305) further comprises an insulating material or a reflective material disposed between at least two layers of the warming blanket.

12. The warming blanket (100, 200, 300) of any of claims 8-11, wherein each of the one or more pockets (110, 210, 310) further comprises at least one of the following: an elastic material; or a material that controls the flow of air to a heating element inserted into the pocket.

13. The warming blanket (100, 200, 300) of any of claims 8-12, wherein the warming blanket further comprises a weight (115) sufficient to retain the warming blanket against a user's body when place on top of a user.

14. The warming blanket (100, 200, 300) of any of claims 8-13, wherein the warming blanket is enclosed within a substantially air tight polymer packaging.

## Patentansprüche

1. Verfahren zur Verwendung einer Wärmedecke (100, 200, 300), wobei das Verfahren die Schritte umfasst:
Bereitstellen einer Wärmdecke mit einem Hauptteil (105, 205, 305) und einer oder mehreren Taschen (110, 210, 310), die mit dem Hauptteil auswechselbar verbunden sind, wobei jede der einen oder mehreren Taschen ein Heizelement aufweist;
und,
Anbringen der Wärmedecke auf einem Benutzer;
**gekennzeichnet durch** den Schritt:
auswechselbares Einsetzen des Heizelements in eine oder mehrere der Taschen (110, 210, 310), so dass das Heizelement ersetzt werden kann.

2. Verfahren nach Anspruch 1, des Weiteren umfassend, dass die Wärmedecke (100, 200, 300) Luft ausgesetzt wird.

3. Verfahren nach Anspruch 1 oder 2, bei dem die Positionen der Taschen (110, 210, 310) für eine Wärmeverteilung sorgen, die eine angemessene warme Behaglichkeit für den Benutzer bewirkt und noch dazu gleichzeitig einen chirurgischen Zugang zum Benutzer zulässt.

4. Verfahren nach einem vorhergehenden Anspruch, bei dem die Wärmedecke (110, 210, 310) an dem Benutzer anliegend entweder in einem voroperativen Raum oder einem postoperativen Raum angebracht wird.

5. Verfahren nach einem vorhergehenden Anspruch, bei dem die Wärmedecke (110, 210, 310) ungefähr zwanzig Minuten vor deren Anbringen am Benutzer anliegend der Luft ausgesetzt wird.

6. Verfahren nach einem vorhergehenden Anspruch, bei dem die Wärmedecke (110, 210, 310) durch eine wärmeabgebende Reaktion in der chemischen Verbindung in jedem Heizelement selbst erwärmt wird, und bei dem die wärmeabgebende Reaktion ungefähr zwölf Stunden dauert.

7. Verfahren nach einem vorhergehenden Anspruch, bei dem nach Aktivierung von mindestens einem Heizelement die Wärmedecke (110, 210, 310) in einer relativ luftdichten Hülle hermetisch abgeschlossen wird, um die wärmeabgebende Reaktion des mindestens einen Heizelements zu unterdrücken.

8. Wärmedecke (110, 210, 310), umfassend:
einen Hauptteil (105, 205, 305); und
eine oder mehrere Taschen (110, 210, 310), die auswechselbar mit dem Hauptteil verbindbar sind, bei der jede der einen oder mehreren Taschen ein entsprechendes Heizelement halten soll;
**dadurch gekennzeichnet, dass**:
eine oder mehrere der Taschen (110, 210, 310) so angepasst sind, dass das Heizelement auswechselbar in die Tasche einsetzbar ist, wobei:
A) die Tasche einen einzelnen Hüllenabschnitt aufweist, der den auswechselbaren Einsatz des Heizelements erleichtert; oder
B) die Tasche ein elastisches oder dehnbares Material aufweist, das gedehnt werden kann, um das Heizelement innerhalb der Tasche anzuordnen; oder
C) die Tasche einen ersten Abschnitt (405) und einen zweiten Abschnitt (410) umfasst, wobei:
i) das Heizelement zwischen dem ersten Abschnitt (405) und dem zweiten Abschnitt (410) einsetzbar ist; oder
ii) der erste Abschnitt (405) und/oder der zweite Abschnitt (410) biegsam sind, wodurch der Einsatz des Heizelements erlaubt wird.

9. Wärmedecke (100, 200, 300) nach Anspruch 8, umfassend Wärmeelemente, bei der jedes der entsprechenden Wärmeelemente zumindest eines von Folgendem aufweist: Kohlenstoff, Holzkohle, Vermiculit oder eine durch Luft aktivierte chemische Verbindung, eine chemische Verbindung mit wärmeabgebender Reaktion bei Aktivierung durch Aussetzung der Luft, eine genügende Menge und Verteilung der chemischen Verbindung, so dass die wärmeabgebende Reaktion nach der Aussetzung der Luft ungefähr zwölf Stunden dauert, eine wärmeabgebende Reaktion, die ungefähr zwanzig Minuten nach der Aussetzung von Luft beginnt, oder eine Reaktionszeit zum Bereitstellen von Wärme ungefähr für eine Zeit des davor liegenden Aufnahmevorgangs eines Benutzers.

10. Wärmedecke (100, 200, 300) nach Anspruch 8 oder 9, bei der der Hauptteil (105, 205, 305) ein beliebiges von Folgendem aufweist: eine im Wesentlichen rechteckige Form, eine im Wesentlichen quadratische Form, eine im Wesentlichen elliptische Form, eine im Wesentlichen kreisförmige Form, die Form eines Kleidungsstücks, eine Form, die im Wesentlichen der Form eines menschlichen Körpers entspricht; einen oder mehrere herausgeschnittene Teile zum Unterbringen von einem oder mehreren entsprechenden Anhängen des menschlichen Körpers; ein wasserbeständiges oder wasserdichtes Material; oder zumindest ein Reflexionsmaterial.

11. Wärmedecke (100, 200, 300) nach einem der Ansprüche 8 bis 10, bei der der Hauptteil (105, 205, 305) des Weiteren ein isolierendes Material oder ein reflektierendes Material aufweist, das zwischen mindestens zwei Lagen der Wärmedecke angeordnet ist.

12. Wärmedecke (100, 200, 300) nach einem der Ansprüche 8 bis 11, bei der jede der einen oder mehreren Taschen (110, 210, 310) des Weiteren mindestens eines von Folgendem aufweist: ein elastisches Material; oder ein Material, das den Luftstrom zu einem in die Tasche eingesetzten Heizelement regelt.

13. Wärmedecke (100, 200, 300) nach einem der Ansprüche 8 bis 12, wobei die Wärmedecke außerdem ein Gewicht (115) aufweist, das ausreichend ist, um die Wärmedecke an dem Körper eines Benutzers zu halten, wenn sie sich auf der Oberseite eines Benutzers befindet.

14. Wärmedecke (100, 200, 300) nach einem der Ansprüche 8 bis 13, wobei die Wärmedecke innerhalb einer im Wesentlichen luftdichten Polymerverpackung eingefasst ist.

## Revendications

1. Procédé d'utilisation d'une couverture chauffante (100, 200, 300), le procédé comprenant les étapes de :
fournir une couverture chauffante avec un corps (105, 205, 305) et une ou plusieurs poches (110, 210, 310) reliées au corps de manière amovible, chacune de la ou des poches comprenant un élément chauffant ;
et,
positionner la couverture chauffante sur un utilisateur ;
**caractérisé par** l'étape de :
insérer ledit élément chauffant de manière amovible dans une ou plusieurs desdites poches (110, 210, 310) de sorte que l'élément chauffant peut être remplacé.

2. Procédé selon la revendication 1, comprenant en outre l'exposition de la couverture chauffante (100, 200, 300) à l'air.

3. Procédé selon les revendications 1 ou 2, dans lequel les positions desdites poches (110, 210, 310) permettent une répartition de chaleur qui assure un confort thermique adapté à l'utilisateur en permettant toutefois simultanément un accès chirurgical à l'utilisateur.

4. Procédé selon l'une quelconque des revendications précédentes, dans lequel la couverture chauffante (110, 210, 310) est positionnée à proximité de l'utilisateur dans une salle pré-opératoire ou une salle postopératoire.

5. Procédé selon l'une quelconque des revendications précédentes, dans lequel la couverture chauffante (110, 210, 310) est exposée à l'air approximativement vingt minutes avant de positionner la couverture chauffante à proximité de l'utilisateur.

6. Procédé selon l'une quelconque des revendications précédentes, dans lequel la couverture chauffante (110, 210, 310) est auto-chauffée par une réaction exothermique dans la composition chimique de chaque élément chauffant, et dans lequel la réaction exothermique dure approximativement douze heures.

7. Procédé selon l'une quelconque des revendications précédentes, dans lequel après activation d'au moins un élément chauffant, la couverture chauffante (110, 210, 310) est étanchéifiée dans une enceinte relativement étanche à l'air pour inhiber la réaction exothermique de l'au moins un élément chauffant.

8. Couverture chauffante (110, 210, 310), comprenant :
un corps (105, 205, 305) ; et
une ou plusieurs poches (110, 210, 310) pouvant être reliées au corps de manière détachable, dans lequel chacune de la ou des poches est adaptée pour contenir un élément chauffant respectif ;
**caractérisée en ce que** :
une ou plusieurs des poches (110, 210, 310) sont adaptées de sorte que ledit élément chauffant peut être inséré dans la poche de manière amovible, dans lequel :
A) la poche comprend une partie de manchon unique qui facilite l'insertion amovible de l'élément chauffant ; ou,
B) la poche comprend un matériau élastifié ou élastique qui peut être étiré afin de placer l'élément chauffant à l'intérieur de la poche ; ou,
C) la poche comprend une première partie (405) et une seconde partie (410), dans laquelle :
i) l'élément chauffant peut être inséré entre la première partie (405) et la seconde partie (410) ; ou,
ii) la première partie (405) et/ou la seconde partie (410) sont souples, permettant ainsi l'insertion de l'élément chauffant.

9. Couverture chauffante (100, 200, 300) selon la revendication 8, comprenant des éléments chauffants, dans laquelle chacun des éléments chauffants respectifs comprend au moins un des éléments suivants : carbone, charbon, vermiculite, ou une composition chimique activée par l'air, une composition chimique ayant une réaction exothermique lorsqu'elle est activée par une exposition à l'air, une quantité suffisante et une répartition de la composition chimique de sorte que la réaction exothermique dure approximativement douze heures après l'exposition à l'air, une réaction exothermique débutant approximativement vingt minutes après l'exposition à l'air, ou un temps de réaction pour fournir de la chaleur approximativement à un moment de la pré-procédure d'admission d'entrée de l'utilisateur.

10. Couverture chauffante (100, 200, 300) selon les revendications 8 ou 9, dans laquelle le corps (105, 205, 305) comprend l'une quelconque des formes suivantes : une forme sensiblement rectangulaire, une forme sensiblement carrée, une forme sensiblement elliptique, une forme sensiblement circulaire, une forme de vêtements, une forme qui s'adapte sensiblement à la forme d'un corps humain ; une ou plusieurs parties découpées pour recevoir un ou plusieurs appendices respectifs du corps humain ; un matériau résistant à l'eau ou étanche à l'eau ; ou au moins un matériau réfléchissant.

11. Couverture chauffante (100, 200, 300) selon l'une quelconque des revendications 8 à 10, dans laquelle le corps (105, 205, 305) comprend en outre un matériau isolant ou un matériau réfléchissant disposé entre au moins deux couches de la couverture chauffante.

12. Couverture chauffante (100, 200, 300) selon l'une quelconque des revendications 8 à 11, dans laquelle chacune de la ou des poches (110, 210, 310) comprend en outre au moins un des matériaux suivants : un matériau élastique ; ou un matériau qui régule l'écoulement d'air jusqu'à un élément chauffant inséré dans la poche.

13. Couverture chauffante (100, 200, 300) selon l'une quelconque des revendications 8 à 12, dans laquelle la couverture chauffante comprend en outre un poids (115) suffisant pour maintenir la couverture chauffante contre le corps d'un utilisateur lorsqu'elle est placée sur un utilisateur.

14. Couverture chauffante (100, 200, 300) selon l'une quelconque des revendications 8 à 13, dans laquelle la couverture chauffante est enfermée à l'intérieur d'un emballage polymère sensiblement étanche à l'air.
